# EUROPEAN PATENT APPLICATION

(11) **EP 3 639 753 A1**
(43) Date of publication of application: **22.04.2020**
(21) Application number: 19203943.6
(22) Date of filing: 17.10.2019
(51) Int. Cl.: A61B 8/08

(54) **ULTRASOUND IMAGING APPARATUS AND METHOD OF CONTROLLING THE SAME**

(30) Priority: 17.10.2018 KR 20180123622
(71) Applicant: Samsung Medison Co., Ltd., Hongcheon-gun, Gangwon-do, 25108 (KR)
(72) Inventor: Kim, Mose, 07431 Seoul (KR); Lee, Yoon Chang, 10415 Gyeonggi-do (KR)
(74) Representative: Jacobs, Bart

(57) **Abstract**

Provided is an ultrasound imaging apparatus including: an ultrasound probe configured to transmit an ultrasound signal to an object and receive an eco-signal from the object; a contrast agent destructor configured to transmit acoustic energy for destructing the contrast agent introduced into the object; and a controller configured to acquire a first image including a contrast agent signal and a tissue signal on the basis of the received echo signal before the destruction of the contrast agent introduced into the object, acquire a second image including the tissue signal on the basis of the echo signal after the destruction of the contrast agent introduced into the object, and generate a third image on the basis of a subtraction between the first image and the second image.

## Description

### CROSS-REFERENCE TO RELATED APPLICATION

This application is based on and claims priority under 35 U.S.C. §119 to Korean Patent Application No. 10-2018-0123622, filed on October 17, 2018 in the Korean Intellectual Property Office, the disclosure of which is incorporated herein by reference.

The disclosure relates to an ultrasound imaging apparatus for acquiring an internal image of an object using ultrasound and a method of controlling the same.

An ultrasound imaging apparatus radiates ultrasound signals generated from a transducer of a probe to an object, and acquires an image regarding the inside of the object using information about echo signals reflected from the object. Here, the acquired image may be generated based on various reflection signals reflected from a tissue of the object, a space between the tissues, and a foreign substance.

Recently, contrast agents have been used for ultrasound imaging apparatuses. The contrast agent is injected into the object to improve the contrast of the tissue and the space between tissues such that a precise ultrasound image is provided.

Therefore, it is an object of the present disclosure to provide an ultrasound imaging apparatus and a method of controlling the same, capable of effectively removing a noise signal from a contrast agent image without acquiring a separate tissue image.

Additional aspects of the invention will be set forth in part in the description which follows and, in part, will be obvious from the description, or may be learned by practice of the invention.

In accordance with one aspect of the present invention, an ultrasound imaging apparatus includes: a probe configured to receive a signal regarding an object; and a controller configured to acquire a first image that corresponds to an image before a contrast agent introduced into the object is destructed and includes a contrast agent signal and a tissue signal, acquire a second image that corresponds to an image after the contrast agent is destructed and includes the tissue signal, and generate a third image on the basis of a subtraction between the first image and the second image.

The third image may be an image in which the tissue signal and at least part of a noise signal included in the first image are removed and the contrast agent signal is included.

The contrast agent destructor may be provided on at least one of the ultrasound probe, a main body, or an outside of the main body.

The contrast agent destructor may adjust at least one of a transmission voltage, a transmission frequency, a diameter of a transmission area, a pattern of the transmission area or a focus of the transmission area, of the acoustic energy.

The pattern of the transmission area may be provided such that a piezoelectric member in the ultrasound probe generates acoustic energy in units of at least one element.

The controller may perform an operation using at least one of an arithmetic operation, a logical operation, or a mapping using a lookup table, between the first image and the second image.

The controller may generate the third image as a result of a subtraction for excluding the tissue signal corresponding to the second image from the first image.

The controller may be configured to: perform a subtraction between the first image and the second image; wherein the first image includes a first contrast agent signal, a second contrast agent signal, a tissue signal, and a noise signal; the second image includes a residual signal of the second contrast agent signal that is left as being incompletely removed by the transmitted acoustic energy, the tissue signal, and the noise signal; as a result of the subtraction between the first image and the second image, the third image in which the tissue signal and at least part of the noise signal are removed and the first contrast agent signal and at least part of the second contrast agent signal are included.

The controller may be configured to correct the part of the second contrast agent signal included in the third image to match the first contrast agent signal included in the third image.

The controller may be configured to generate a fourth image by adding a signal strength corresponding to the third image to the first image.

The controller may be configured to perform motion correction to increase a correlation between the first image and the second image before the performing of the operation.

The controller may be configured to set a weight on at least one of the first image or the second image, and adjust the weight.

The controller may be configured to adjust signal strength of a contrast agent signal included in the fourth image by offsets and random N values

In accordance with another aspect of the present invention, a method of an ultrasound imaging apparatus includes: acquiring a first image including a tissue signal of an object into which a contrast agent is introduced and a contrast agent signal; transmitting acoustic energy for destructing the contrast agent to the object and acquiring a second image in which the contrast agent signal is attenuated; and generating a third image as a result of a subtraction between the first image and the second image.

The generating of the third image may include generating the third image as a result of a subtraction for excluding the tissue signal corresponding to the second image and a noise signal from the first image.

The generating of the third image may include: performing a subtraction between the first image and the second image, wherein the first image includes a first contrast agent signal, a second contrast agent signal, a tissue signal, and an a noise signal, the second image includes a residual signal of the second contrast agent signal that is left as being incompletely removed by the transmitted acoustic energy, the tissue signal, and the noise signal; and as a result of the subtraction between the first image and the second image, generating the third image in which the tissue signal and at least part of the noise signal are removed and the first contrast agent signal and at least part of the second contrast agent signal are included.

The generating of the third image may include correcting the part of the second contrast agent signal included in the third image to match the first contrast agent signal included in the third image.

The generating of the third image may include generating a fourth image by adding a signal strength corresponding to the third image to the first image.

The generating of the third image may include performing motion correction to increase a correlation between the first image and the second image before the performing of the operation.

The generating of the third image may include setting a weight on at least one of the first image or the second image, and adjusting the weight.

### BRIEF DESCRIPTION OF THE DRAWINGS

These and/or other aspects of the invention will become apparent and more readily appreciated from the following description of the embodiments, taken in conjunction with the accompanying drawings of which:
FIGS. 1A and 1B are views for describing a contrast agent diagnostic mode of an ultrasound imaging apparatus;
FIG. 2 is a view for describing a process of acquiring a contrast agent image;
FIG. 3 is an external view of an ultrasound imaging apparatus, according to one embodiment;
FIG. 4 is a control block diagram illustrating an ultrasound imaging apparatus according to one embodiment;
FIG. 5 is an external view of an ultrasound probe according to one embodiment;
FIG. 6 is an external view of an ultrasound probe according to another embodiment;
FIG. 7 is a view for describing a process of acquiring an ultrasound image according to one embodiment;
FIG. 8 is a view for describing a process of acquiring an ultrasound image according to another embodiment;
FIG. 9 is a view for describing a process of acquiring an ultrasound image according to another embodiment; and
FIG. 10 is a flowchart showing a method of controlling an ultrasound imaging apparatus, according to one embodiment.

### DETAILED DESCRIPTION

Hereinafter, embodiments of the disclosure will be described in detail with reference to the accompanying drawings. Advantages and features of the disclosure, and methods of achieving the same will be clearly understood with reference to the accompanying drawings and the following detailed embodiments. However, the inventive concept is not limited to embodiments described herein, but may be implemented in various different forms. Rather, these embodiments are provided so that this disclosure is thorough and complete and fully conveys the inventive concept to those skilled in the art, and the scope of the inventive concept is defined by the appended claims. Like numerals refer to like elements throughout the specification.

Unless otherwise defined, all terms including technical and scientific terms used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein. The terminology used herein is for the purpose of describing particular embodiments only and is not intended to be limiting of the disclosure. As used herein, the singular forms "a," "an," and "the" are intended to include the plural forms as well, unless the context clearly indicates otherwise.

It will be further understood that the terms "comprises," "comprising," "includes," and/or "including," when used herein, specify the presence of stated features, integers, steps, operations, elements, and/or components but do not preclude the presence or addition of one or more other features, integers, steps, operations, elements, components, and/or groups thereof.

Before providing a detailed description of the disclosure, terms used herein will be clarified.

An "object" may include a human or animal, or a portion of a human or animal. For example, the object may include organs, such as the liver, heart, uterus, brain, breast, and abdomen, or blood vessels.

In addition, throughout the specification, an "ultrasound image" refers to an image of an object acquired using ultrasound. In this case, the ultrasound image may be a two-dimensional (2D) or a three-dimensional (3D) image. Also, the ultrasound image may include a tissue image showing an anatomical structure of a test portion of an object and a contrast agent image showing a contrast agent of the test portion.

An ultrasound contrast agent (hereinafter, referred to as a contrast agent) enhances echo signals in a location in which ultrasound images are difficult to be acquired due to weak echo signals, for example, blood vessels located deep in an object, small lesions, or the like. The contrast agent is injected into the blood vessel of the object and travels within the object along the vessel. When irradiated with ultrasound, the contrast agent makes a non-linear motion or collapses, causing backscattering. The ultrasound imaging apparatus may generate a contrast agent image using such backscattering.

In detail, the contrast agent may be divided a microparticle contrast agent and a nanoparticulate contrast agent depending on the particle sizes. For example, the microparticle contrast agent may represent microbubbles. The microbubbles may have a size of 1 µm to 4 µm. The microbubbles may be composed of a phospholipid membrane that surrounds gas, such as perfluorocarbon (PFC). In addition, the nanoparticle contrast agent may represent PFC nanodroplets, or polyactic acid (PLA) nanobubbles. The PFC nanodroplets may have a size of 200 nm to 400nm, and the PLA nanobubbles may have a size of 40 nm to 200nm.

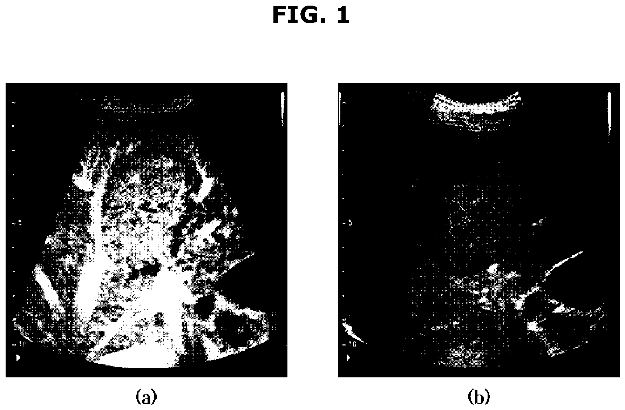

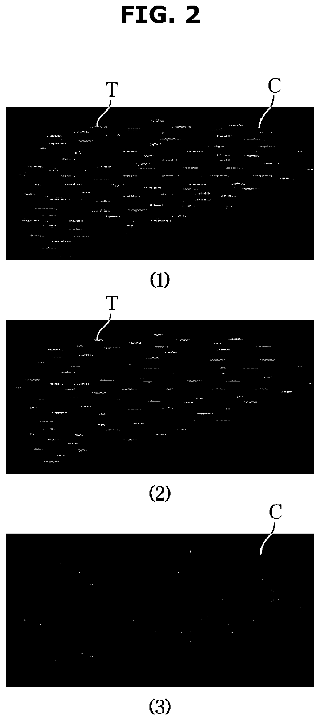

Hereinafter, the operating principles and embodiments of the disclosure will be described with reference to the accompanying drawings. First, a method of generating a general contrast agent image will be described with reference to FIGS. 1 and 2.

FIGS. 1A and 1B are views illustrating general ultrasound images in a contrast agent diagnostic mode. FIG. 1A shows an image represented mainly by a contrast agent signal, and FIG. 1B shows an image represented mainly by a tissue signal. Here, each image may simultaneously output the contrast agent signal and the tissue signal, but the signals in each image differ in strength and distribution, and therefore the output images have different characteristics. In addition, the tissue signal is shown at the same brightness without change over time, while the contrast agent signal changes in brightness over time.

The contrast agent image is implemented using characteristics in which a contrast agent composed of microbubbles generates a greater response signal than with a general tissue even at a low transmission acoustic pressure, so that the contrast agent signal is generated at the most even with a low transmission acoustic pressure while minimizing the tissue signal. Accordingly, the contrast agent image shows significantly different characteristics depending on the presence or absence of the contrast agent. However, the contrast agent image, before the contrast agent is injected or after the contrast agent is extinguished, only shows a black background or part of the tissue signal due to insufficiency of the signals, which causes difficulty in specifying the location of a lesion only using the contrast agent image. Here, one way used to identify the location of a lesion is to provide a contrast agent image and a tissue image synchronized with the contrast agent image side by side as shown in FIGS. 1A and 1B, but the method incurs waste of cost and time caused by providing a transmission and reception device for the separate tissue image.

On the other hand, the contrast agent image includes not only contrast agent signals but also tissue signal components that are not removed at low transmission acoustic pressures, which results in degradation of diagnostic performance.

One way to remove a tissue signal and image a contrast agent signal is to use a differential image between a contrast agent image (1) and a tissue image (2) as shown in FIG. 2. Since the tissue signals included in the contrast agent image are anatomically identical to the tissue signals in the tissue image, the subtraction results in the same effect of removing the tissue signals.

However, the above-described method requires a separate tissue image to be referred for a tissue signal, and also requires a transmission/reception technique for showing the separate tissue image and a cost and time for constructing the image. In addition, even when such a tissue image exists, since the characteristics of a tissue signal shown in the tissue image are different from those of a tissue signal shown in the contrast agent image, the efficiency of removing the tissue signal is not superior. In terms of image characteristics, the contrast agent image is represented by distinguishing the contrast agent signal from the tissue signal through a specifically designed transmission/reception technique, while the tissue image is represented based on brightness without distinguishing the contrast agent signal and the tissue signal. Since the tissue image includes not only a tissue signal but also a contrast agent signal, when the tissue image is directly used to acquire a difference image with the contrast agent image, the contrast agent signal is also caused to be removed together with the tissue signal.

The disclosure is provided to obviate the above-described limitations shown in FIGS. 1A, 1B, and 2 by removing at least a part of the tissue signals which inhibits the diagnosis in the contrast agent image. In detail, according to the disclosure, high acoustic energy for destructing a contrast agent is applied to an object to acquire that a tissue image in which only the contrast agent signal is removed, so that a contrast agent image outputting only a diagnosis portion is acquired.

Hereinafter, an embodiment of an ultrasound apparatus and a method of controlling the ultrasound apparatus according to one aspect will be described in detail with reference to the accompanying drawings.

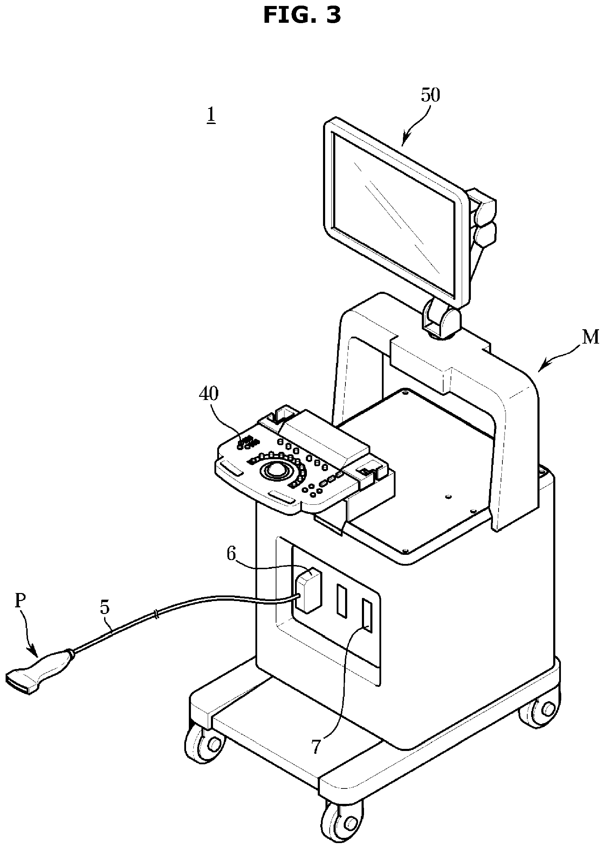

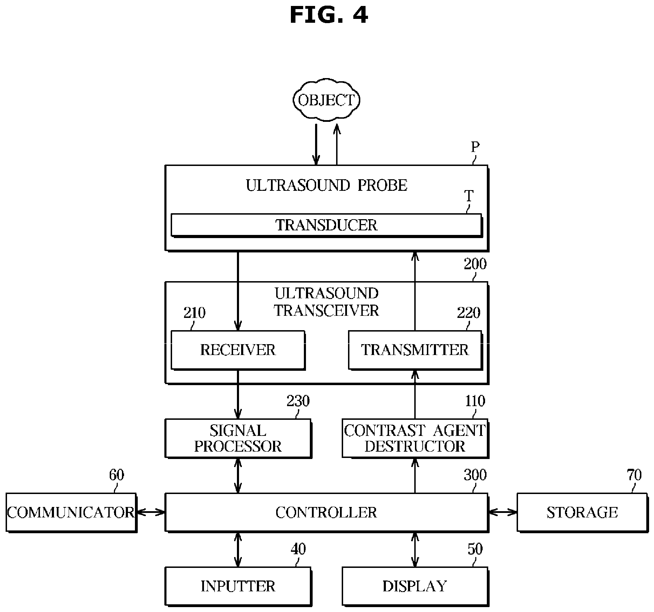

FIG. 3 is an external view of an ultrasound imaging apparatus 1, according to an embodiment, and FIG. 4 is a control block diagram illustrating the ultrasound imaging apparatus according to an embodiment.

Referring to FIG. 3, the ultrasound imaging apparatus 1 includes: an ultrasound probe P configured to transmit ultrasound to an object, receive an ultrasound echo signal from the object, and convert the received ultrasound echo signal into an electrical signal; and a main body M connected to the ultrasound probe P and having an inputter 40 and a display 50 and configured to display an ultrasound image.

The ultrasound probe P is connected to the main body M of the ultrasound imaging apparatus 1 through a cable 5 to receive various signals required for controlling the ultrasound probe P, or transmit an analog signal or digital signal corresponding to the ultrasound echo signal received by the ultrasound probe P to the main body M. However, the embodiment of the ultrasound probe P is not limited thereto, and the ultrasound probe P may be implemented as a wireless probe to transmit and receive signals through a network formed between the ultrasound probe P and the main body M. In addition, the ultrasound probe P or the main body M may be provided with a contrast agent destructor 110 to transmit acoustic energy for destructing a contrast agent introduced into an object.

The contrast agent destructor 110 may destruct microbubbles constituting a contrast agent introduced into an object by a mechanical action of an ultrasound acoustic pressure. Meanwhile, the contrast agent destructor 110 may adjust the value of the acoustic energy by adjusting at least one of a transmission voltage, a transmission frequency, a diameter of a transmission area, a pattern of the transmission area, and a focus of the transmission area, of the acoustic energy.

In addition, the pattern of the transmission area may be designed to generate acoustic energy in units of at least one element. In detail, the pattern of the transmission area may be designed such that a piezoelectric member provided in the ultrasound probe P generates a transmission signal in units of at least one element.

The cable 5 is connected at one end to the ultrasound probe P and is provided at the other end with a connector 6 that is coupled to or separated from in a slot 7 of the main body M. The main body M and the ultrasound probe P may exchange control commands or data using the cable 5. For example, when a user inputs information about a focal depth, a size or shape of an aperture, or a steering angle through the inputter 40, the information is transmitted to the ultrasound probe P through the cable 5 to thereby be used by a beamforming apparatus (not shown).

Alternatively, when the ultrasound probe P is implemented as a wireless probe as described above, the ultrasound probe P is connected to the main body M through a wireless network, rather than the cable 5. Even when the main body M is connected to the main body M through a wireless network, the main body M and the ultrasound probe P may exchange the above-described control commands or data.

Referring to FIG. 4, the ultrasound probe P may include a transducer T, and the main body M may include a contrast agent destructor 110, an ultrasound transceiver 200, a signal processor 230, a controller 300, a manipulation panel 40, a display 50, a communicator 60, and a storage 70.

Referring to the configuration of the main body M, the controller 300 may control overall operations of the ultrasound imaging apparatus 1 and perform overall operations of processing the acquired image. In detail, the controller 300 may control the contrast agent destructor 110, the ultrasound transceiver 200, the signal processor 230, the display 50, and the like.

The signal processor 230 may divide ultrasound signals focused through the ultrasound probe P on the basis of signal characteristics. For example, the signal processor 230 may distinguish a tissue signal reflected by an object, a noise signal, and a contrast agent signal on the basis of the signal characteristics. In addition, the signal processor 230 may generate a coherent two-dimensional image or three-dimensional image with respect to an object portion inside the object on the basis of the classified ultrasound signals.

In addition, the signal processor 230 may convert coherent image information into ultrasound image information according to a diagnosis mode, such as a brightness mode (B-mode) or a Doppler mode (D-mode). For example, when a diagnostic mode is set to the B-mode, the signal processor 230 may perform an A/D conversion process or the like and generate ultrasound image information for a B-mode image in real time.

When a photography mode is set to the D-mode, the signal processor 230 extracts phase change information from ultrasound signals, and calculates information about blood flow corresponding to each point of a photographing section, such as speed, power, and distribution, and generates ultrasound image information for a D-mode image in real time.

The inputter 40 may receive a user's instruction or command, and the controller 300 may control the ultrasound imaging apparatus 1 according to the command input by the user. The user inputs an ultrasound diagnosis start command, a diagnostic mode selection command to select a diagnosis mode, such as an amplitude mode (A-mode), a brightness mode (B-mode), a color mode, a Doppler mode (D-mode) and a motion mode (M-mode), or region of interest (ROI) setting information including the size and location of an ROI through the inputter 40.

The inputter 40 may include various devices for inputting data, instructions, or commands by a user, such as a keyboard, a mouse, a trackball, a tablet PC, or a touch screen module.

The display 50 may show a menu or guide required for ultrasound diagnosis and an ultrasound image acquired during an ultrasound diagnosis process. The ultrasound image shown on the display 50 may be an A-mode ultrasound image or a B-mode ultrasound image, or may be a 3D stereoscopic ultrasound image. The display 50 may be implemented using generally known display methods, such as a cathode ray tube (CRT) and a liquid crystal display (LCD). Here, the display 50 may show a third image in which a tissue signal is removed and a contrast agent signal is output. Detailed features of the third image will be described below.

Hereinafter, the configuration of the ultrasound probe P will be described in detail. A transducer array TA is provided at the end of the ultrasound probe P. The ultrasound transducer array TA refers to a plurality of ultrasound transducer elements arranged in an array.

In this case, the ultrasound probe P may include a transducer for conversion between an electrical signal and an ultrasound signal to transmit ultrasound to the inside of the object. The transducer array may be implemented as a one-dimensional or two-dimensional transducer array, and the transducer array is composed of a plurality of transducer elements.

The main body M includes the contrast agent destructor 110, and the contrast agent destructor 110 transmits a signal for adjusting at least one of a magnitude, a frequency, or a pattern of the voltage of a transmitter 220 to the ultrasound probe P. Accordingly, the ultrasound probe P may destruct the contrast agent introduced into the object even during the scanning for diagnosing the object. The contrast agent destructor 110 may be provided in the ultrasound probe P or the main body M. The contrast agent destructor 110 may be a device separated from the ultrasound imaging apparatus 1 and having a function of transmitting acoustic energy to the object.

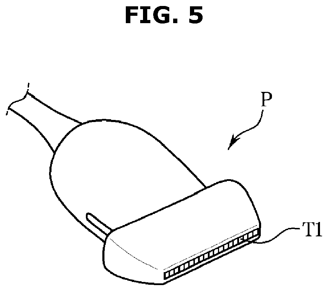

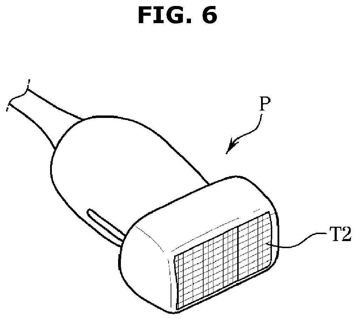

For example, the transducer may include a one-dimensional array transducer T1 as shown in FIG. 5. For another example, the transducer may include a two-dimensional array transducer T2 as shown in FIG. 6.

For example, each transducer element constituting the one-dimensional array transducer may perform conversion between an ultrasound signal and an electrical signal. For this, the transducer element may be implemented using a magnetostrictive ultrasound transducer using a magnetostrictive effect of a magnetic body, a piezoelectric ultrasound transducer using a piezoelectric effect of a piezoelectric material, or a piezoelectric micromachined ultrasound transducer (pMUT), or may be implemented using a capacitive micromachined ultrasound transducer (hereinafter, referred to as cMUT) transmitting and receiving ultrasound using vibration of hundreds or thousands of thin films that are microfabricated.

On the other hand, the transducer elements may be arranged in the form of a straight line, or in the form of a convex. In both cases, the basic operation principle of the ultrasound probe P is the same, but when the ultrasound probe P has transducer elements arranged in the form of a convex, since ultrasound waves radiated from the transducer form a fan-shape, a fan-shaped ultrasound image is generated.

Referring to FIG. 6, the transducer of the ultrasound probe P may include the 2D transducer array T2 as described above. When the transducer includes the 2D transducer array T2, the inside of the object may be imaged in a three dimension.

Since each transducer element constituting the two-dimensional array transducer is the same as the transducer element constituting the one-dimensional transducer array, detailed description thereof will be omitted.

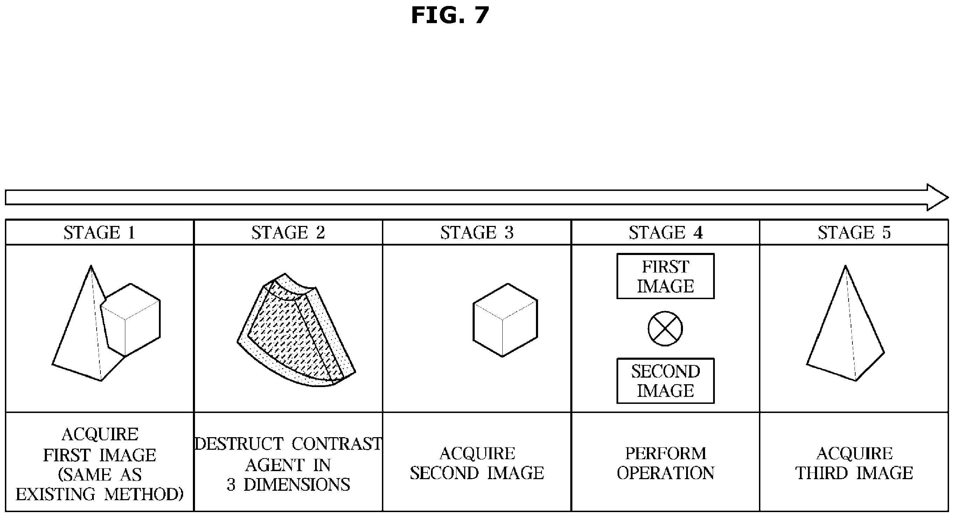

FIG. 7 is a view for describing a process of acquiring an ultrasound image according to an embodiment. The image generated according to the embodiment is an image in which a tissue signal T and a noise signal N are removed through a subtraction between an image before contrast agent destruction and an image after contrast agent destruction.

A first image refers to a general contrast agent image. In detail, the first image is an image acquired before a contrast agent introduced into an object is destructed. Here, the first image does not only include a contrast agent signal C but also includes a tissue signal T and a noise signal N that are not removed at low transmission acoustic pressure. However, the tissue signal T and the noise signal N, which are not clearly distinguished from the contrast agent signal C, may cause diagnostic performance to be deteriorated.

Accordingly, the contrast agent destructor may transmit acoustic energy to a portion of the object into which the contrast agent is introduced, to acquire a second image in which the contrast agent signal is removed. The second image is an image acquired after the contrast agent introduced into the object is destructed by the acoustic energy. The second image refers to an image including at least one of the tissue signal T or the noise signal N.

When the first image and the second image are acquired through the above-described process, an operation between the first image and the second image is performed. In detail, the operation between the first image and the second image may represent a subtraction for canceling signals corresponding to each other. Here, the corresponding signal may be at least one of the tissue signal T or the noise signal N. Accordingly, a third image generated as a result of the operation between the first image and the second image mainly outputs the contrast agent signal C.

Meanwhile, the controller may perform various operations to acquire the third image. For example, the third image may be generated by performing an arithmetic operation, a logical operation, and a mapping using a lookup table on the basis of data between the first image and the second image.

In detail, the controller may generate the third image by performing a subtraction for excluding a signal corresponding to a signal in the second image, from the first image. Here, the corresponding signal may refer to at least one of a tissue signal or a noise signal, which is included in both of the first image and the second image. Accordingly, the third image, having the tissue signal and the noise signal removed, may be provided to the display such that only a contrast agent image is visible.

According to the embodiment, the contrast agent destructor may transmit acoustic energy for destructing the contrast agent introduced into the object. Here, the transmitted acoustic energy may vary in value depending on the contrast agent introduced into the object. The controller may generate the third image by performing a subtraction between the first image in which the tissue signal reflected from the object and the contrast agent signal are output and the second image in which the contrast agent signal is attenuated or removed by the transmitted acoustic energy. Here, the third image refers to an image in which only contrast agent signals of interest in the ultrasound diagnosis process are output.

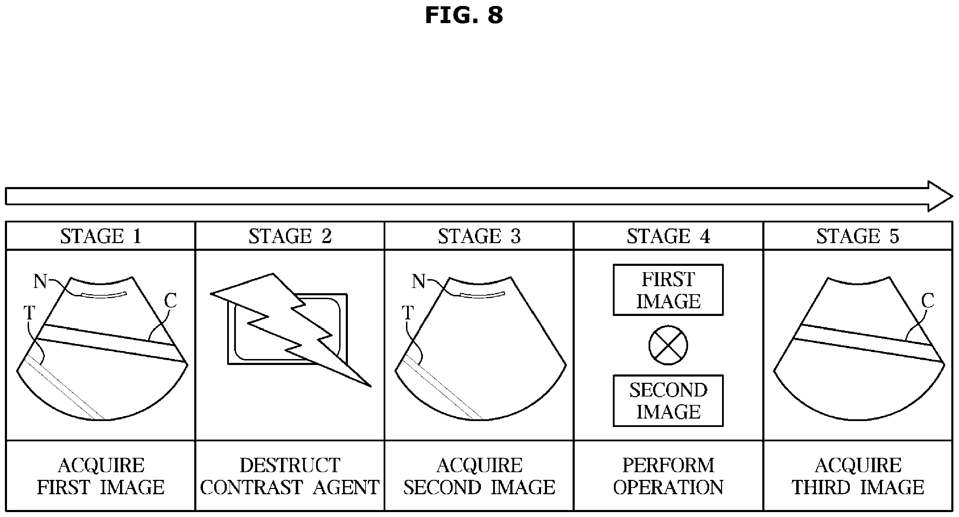

FIG. 8 is a view for describing a process of acquiring an ultrasound image according to another embodiment. FIG. 8 illustrates a process of generating a contrast agent image from a 3D image rather than a 2D image. For example, according to the embodiment, a 3D image of Hysterosalpingo-contrast-sonography (HyCoSy) may provide the user with a diagnostic image with a noise signal and a tissue signal removed such that blockage of fallopian tubes is easily checked..

A first image according to the embodiment corresponds to an image acquired through stereoscopic scanning. Conventionally, a tissue signal and a noise signal in a first image are removed by identify the tissue signal and the noise signal in volume data with naked eyes. However, such a method requires anatomical knowledge of the user, and time consuming task. For example, there is a need for a separate task of checking a tissue signal T output on the first image shown in FIG. 8 and manually removing the tissue signal T.

After the acquisition of the first image in which the contrast agent signal and the tissue signal are output, in order to acquire a second image in which the contrast agent signal is removed, the contrast agent destructor may transmit acoustic energy to an object portion into which the contrast agent has been introduced. In this case, in order to remove the contrast agent distributed in the 3D image, the contrast agent is destructed according to the shape of the object.

When the first image and the second image are acquired through the above-described process, an operation between the first image and the second image is performed. In detail, the operation between the first image and the second image may be a subtraction for canceling signals corresponding to each other. Here, the corresponding signal may be at least one of the tissue signal T or the noise signal N. Accordingly, a third image generated as a result of the operation between the first image and the second image mainly outputs the contrast agent signal C.

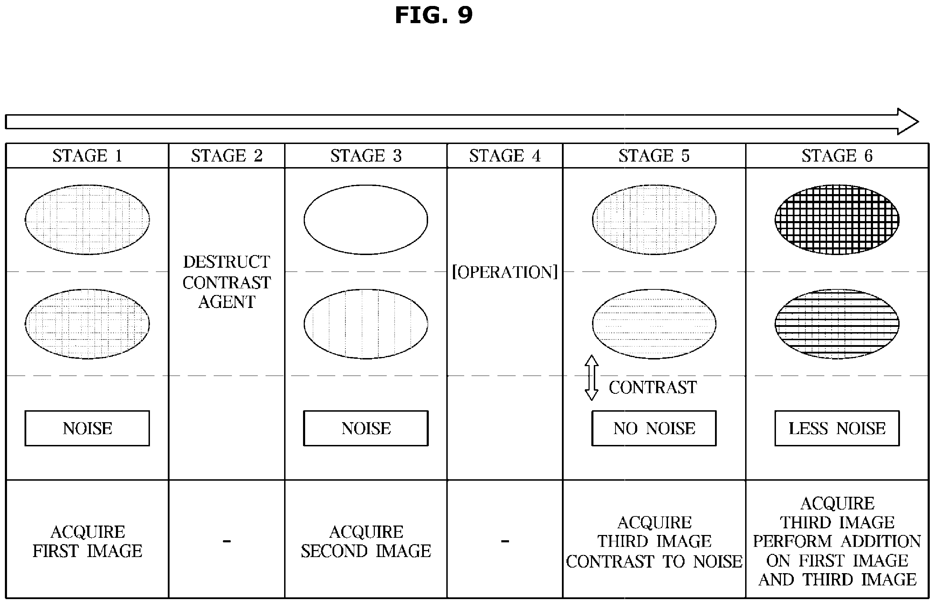

FIG. 9 is a view for describing a process of acquiring an ultrasound image according to another embodiment, in which when some of the contrast agents introduced into the object is incompletely destructed, the brightness of a contrast agent signal is compensated for by performing addition on the original image.

Referring to FIG 9, a first image includes a first contrast agent signal, a second contrast agent signal, and a noise signal. Here, the first contrast agent signal and the second contrast agent signal correspond to contrast agent signals reflected from the same object but distinguished by a slight difference in the degree of destruction of the contrast agent depending on the position.

After the acquisition of the first image, the contrast agent destructor transmits acoustic energy to the object to destruct the contrast agent. In this case, the contrast agent is unevenly destructed with the first contrast agent signal completely removed but the second contrast agent signal incompletely removed, causing a second contrast agent residual signal to be included in a second image.

A third image is derived by performing a subtraction between the first image and the second image. Here, the third image is represented by a contrast agent signal including the first contrast agent signal and the second contrast agent signal except for the second contrast agent residual signal. Since the tissue signal or noise signal included in the first and second images is not destructed by the acoustic energy, the tissue signal or noise signal is not shown in the third image. In other words, the characteristic that the tissue signal and the noise signal are not shown in the third image is used. In this case, the attenuated second contrast agent signal may be corrected according to the first contrast agent signal, so that compensation may be performed even when the contrast agent is incompletely destructed.

According to the embodiment, the controller performs a subtraction between the first image and the second image, and accordingly, generates the third image in which the noise signal is removed and the first contrast agent signal and part of the second contrast agent signal are output. In this case, the first image may include the first contrast agent signal, the second contrast agent signal, and the noise signal, and the second image may include the second contrast agent residual signal incompletely removed by the transmitted acoustic energy and the noise signal. Here, the controller may correct the part of the second contrast agent signal output on the third image to match the strength of the first contrast agent signal output on the third image. Accordingly, even when part of the contrast agent introduced into the object is incompletely destructed, the brightness may be compensated for through the operation process.

In addition, in order to generate a more accurate third image, the controller may set a weight on at least one of the first image or the second image and adjust the weight before performing the subtraction. Here, the weight may be set on the basis of at least one of a tissue signal, a noise signal, or a contrast agent signal included in the image.

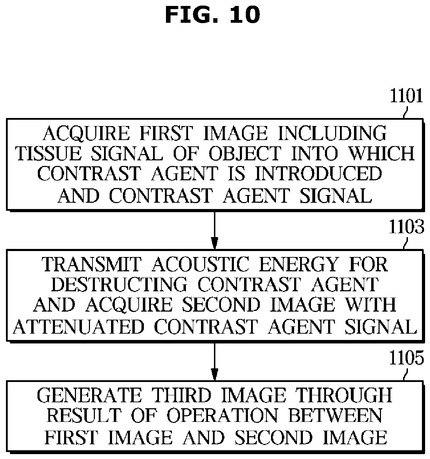

FIG. 10 is a flowchart showing a method of controlling an ultrasound imaging apparatus according to an embodiment. This is merely exemplified for purposes of describing the disclosure, and it should be understood that some operations may be added or omitted when required.

First, a first image including a tissue signal of an object into which a contrast agent is introduced and a contrast agent signal is acquired (1101). The acquired first image may be stored in the storage to perform an operation with a second image in which the contrast agent signal is removed.

Thereafter, the contrast agent destructor transmits acoustic energy for destructing the contrast agent introduced into the object to the object (1103). As for a task of acquiring a 3D image, the scanning for transmitting acoustic energy to an object is performed in three dimensions. As a result, the controller acquires a second image based on the contrast agent destructed by the acoustic energy transmitted to the object. Similar to the first image, the second image may be stored in the storage to thereby be subject to an operation with the first image.

A third image is generated as a result of an operation between the first image and the second image (1105). The third image may be output as a single image on the display.

As is apparent from the above, the ultrasound imaging apparatus and the method of controlling the same can efficiently acquire a contrast agent image required for diagnosis from an ultrasound image without having additional anatomical knowledge.

Although embodiments of the disclosure have been described with reference to the accompanying drawings, a person having ordinary skilled in the art will appreciate that other specific modifications can be easily made without departing from the technical spirit or essential features of the invention. Therefore, the foregoing embodiments should be regarded as illustrative rather than limiting in all aspects.

## Claims

1. An ultrasound imaging apparatus comprising:
an ultrasound probe configured to transmit an ultrasound signal to an object and receive an eco-signal from the object;
a contrast agent destructor configured to transmit acoustic energy for destructing the contrast agent introduced into the object; and
a controller configured to acquire a first image including a contrast agent signal and a tissue signal on the basis of the received echo signal before the destruction of the contrast agent introduced into the object, acquire a second image including the tissue signal on the basis of the echo signal after the destruction of the contrast agent introduced into the object, and generate a third image on the basis of a subtraction between the first image and the second image.

2. The ultrasound imaging apparatus of claim 1, wherein the third image is an image in which the tissue signal and at least part of a noise signal included in the first image are removed and the contrast agent signal is included.

3. The ultrasound imaging apparatus of claim 1 or 2, wherein the contrast agent destructor is provided on at least one of the ultrasound probe, a main body, or an outside of the main body.

4. The ultrasound imaging apparatus of claim 3, wherein the contrast agent destructor adjusts at least one of a transmission voltage, a transmission frequency, a diameter of a transmission area, a pattern of the transmission area or a focus of the transmission area, of the acoustic energy.

5. The ultrasound imaging apparatus of claim 4, wherein the pattern of the transmission area is provided such that a piezoelectric member in the ultrasound probe generates acoustic energy in units of at least one element.

6. The ultrasound imaging apparatus of any one of the preceding claims, wherein the controller performs an operation using at least one of an arithmetic operation, a logical operation, or a mapping using a lookup table, between the first image and the second image.

7. The ultrasound imaging apparatus of any one of the preceding claims, wherein the controller generates the third image as a result of a subtraction for excluding the tissue signal corresponding to the second image from the first image.

8. The ultrasound imaging apparatus of any one of the preceding claims, wherein the controller is configured to:
perform a subtraction between the first image and the second image;
wherein the first image includes a first contrast agent signal, a second contrast agent signal, a tissue signal, and an a noise signal;
the second image includes a residual signal of the second contrast agent signal that is left as being incompletely removed by the transmitted acoustic energy, the tissue signal, and the noise signal;
as a result of the subtraction between the first image and the second image, the third image in which the tissue signal and at least part of the noise signal are removed and the first contrast agent signal and at least part of the second contrast agent signal are included.

9. The ultrasound imaging apparatus of claim 8, wherein the controller is configured to correct the part of the second contrast agent signal included in the third image to match the first contrast agent signal included in the third image.

10. The ultrasound imaging apparatus of claim 8 or 9, wherein the controller is configured to generate a fourth image by adding a signal strength corresponding to the third image to the first image.

11. The ultrasound imaging apparatus of claim 10, wherein the controller is configured to adjust signal strength of a contrast agent signal included in the fourth image by offsets and random N values

12. The ultrasound imaging apparatus of any one of the preceding claims, wherein the controller is configured to perform motion correction to increase a correlation between the first image and the second image before the performing of the operation.

13. The ultrasound imaging apparatus of any one of the preceding claims, wherein the controller is configured to set a weight on at least one of the first image or the second image, and adjust the weight.

14. A method of an ultrasound imaging apparatus, the method comprising:
acquiring a first image including a tissue signal of an object into which a contrast agent is introduced and a contrast agent signal;
transmitting acoustic energy for destructing the contrast agent to the object and acquiring a second image in which the contrast agent signal is attenuated; and
generating a third image as a result of a subtraction between the first image and the second image.

15. The method of claim 14, wherein the generating of the third image comprises generating the third image as a result of a subtraction for excluding the tissue signal corresponding to the second image and a noise signal from the first image.
